(19) **Europäisches Patentamt**
**European Patent Office**
**Office européen des brevets**

(11) **EP 4 172 130 B1**

(12) **EUROPEAN PATENT SPECIFICATION**

(45) Date of publication and mention
of the grant of the patent:
**19.06.2024  Bulletin 2024/25**

(51) International Patent Classification (IPC):
*C07C 1/04* *(2006.01)*    *B01J 29/50* *(2006.01)*
*B01J 29/70* *(2006.01)*    *B01J 29/85* *(2006.01)*
*B01J 35/00* *(2024.01)*

(21) Application number: **21740394.8**

(22) Date of filing: **18.06.2021**

(52) Cooperative Patent Classification (CPC):
(C-Sets available)
**B01J 29/505; B01J 29/50; B01J 29/7015;**
**B01J 29/85; B01J 35/19; C07C 1/0425;**
**C07C 1/043; C07C 1/0435; C07C 1/0445;**
C07C 2521/06; C07C 2523/06; C07C 2523/08;
C07C 2523/10; C07C 2523/80; C07C 2529/50;

(Cont.)

(86) International application number:
**PCT/US2021/038023**

(87) International publication number:
**WO 2022/005769 (06.01.2022 Gazette 2022/01)**

(54) **PROCESSES FOR PREPARING C2 TO C3 HYDROCARBONS**

VERFAHREN ZUR HERSTELLUNG VON C2- BIS C3-KOHLENWASSERSTOFFEN

PROCÉDÉS DE PRÉPARATION D'HYDROCARBURES EN C2 À C3

(84) Designated Contracting States:
**AL AT BE BG CH CY CZ DE DK EE ES FI FR GB**
**GR HR HU IE IS IT LI LT LU LV MC MK MT NL NO**
**PL PT RO RS SE SI SK SM TR**

(30) Priority: **30.06.2020  US 202063045888 P**

(43) Date of publication of application:
**03.05.2023  Bulletin 2023/18**

(73) Proprietor: **Dow Global Technologies LLC**
**Midland, MI 48674 (US)**

(72) Inventors:
• **KIRILIN, Alexey**
**4542 NM Hoek (NL)**
• **MILLAR, Dean, M.**
**Midland, MI 48667 (US)**
• **CHOJECKI, Adam**
**4542 NM Hoek (NL)**
• **DEWILDE, Joseph, F.**
**Collegeville, PA 19426 (US)**
• **POLLEFEYT, Glenn**
**4542 NM Hoek (NL)**
• **NIESKENS, Davy, L.S.**
**4542 NM Hoek (NL)**
• **MALEK, Andrzej**
**Midland, MI 48667 (US)**

(74) Representative: **Boult Wade Tennant LLP**
**Salisbury Square House**
**8 Salisbury Square**
**London EC4Y 8AP (GB)**

(56) References cited:
**WO-A1-2020/005703    US-A1- 2019 336 954**

(52) Cooperative Patent Classification (CPC): (Cont.)
C07C 2529/70; Y02P 20/52

C-Sets
**C07C 1/0425, C07C 9/06;**
**C07C 1/0425, C07C 9/08;**
**C07C 1/0425, C07C 11/04;**
**C07C 1/0425, C07C 11/06;**
**C07C 1/043, C07C 9/06;**
**C07C 1/043, C07C 9/08;**
**C07C 1/043, C07C 11/04;**
**C07C 1/043, C07C 11/06;**

**C07C 1/0435, C07C 9/06;**
**C07C 1/0435, C07C 9/08;**
**C07C 1/0435, C07C 11/04;**
**C07C 1/0435, C07C 11/06;**
**C07C 1/0445, C07C 9/06;**
**C07C 1/0445, C07C 9/08;**
**C07C 1/0445, C07C 11/04;**
**C07C 1/0445, C07C 11/06**

## Description

### BACKGROUND

*Field*

The present specification generally relates to processes that convert a hydrogen and carbon-containing feed stream to $C_2$ and $C_3$ hydrocarbons. In particular, the present specification relates to processes of forming hydrocarbons from a hydrogen and carbon containing feed stream using a hybrid catalyst comprising a metal oxide catalyst component and a microporous catalyst component comprising 8-MR pore openings derived from a natural mineral.

Generally, in hybrid catalyst processes, the hydrogen and carbon-containing feed stream, such as synthesis gas (syngas), comprises hydrogen ($H_2$) gas and a carbon-containing gas. A hybrid catalyst that is used in hybrid catalyst processes generally comprises a combination of a mixed metal oxide component and a microporous catalyst component formulated into a bifunctional catalyst bed that operate in tandem.

*Technical Background*

For a number of industrial applications, hydrocarbons are used, or are starting materials used, to produce plastics, fuels, and various downstream chemicals. $C_2$ to $C_3$ hydrocarbons are particularly useful in downstream applications. A variety of processes for producing these lower hydrocarbons have been developed, including petroleum cracking and various synthetic processes. WO 2020/005703 discloses a synthetically produced molecular sieve having 8-MR pore openings used in the preparation of $C_2$ and $C_3$ hydrocarbons.

Synthetic processes for converting feed carbon to desired products, such as lower hydrocarbons, are known. However, current hybrid catalyst processes typically require a synthetic microporous catalyst component, which may be quite expensive to produce.

Accordingly, a need exists for processes and systems in which the combined $C_2$ and $C_3$ selectivity may be maintained, while decreasing the cost of the microporous catalyst component.

### SUMMARY

Embodiments of the present disclosure meet this need by utilizing syngas to prepare $C_2$ to $C_3$ hydrocarbons with a hybrid catalyst comprising a metal oxide catalyst component and a microporous catalyst component derived from a natural mineral and comprising 8-MR pore openings where the product stream comprises a combined $C_2$ and $C_3$ selectivity greater than 40 carbon mol%. According to one embodiment, a process for preparing $C_2$ to $C_3$ hydrocarbons comprises introducing a feed stream comprising hydrogen gas and a carbon-containing gas selected from the group consisting of carbon monoxide, carbon dioxide, and mixtures thereof into a reaction zone of a reactor, and converting the feed stream into a product stream comprising $C_2$ to $C_3$ hydrocarbons in the reaction zone in the presence of a hybrid catalyst, the hybrid catalyst comprising a metal oxide catalyst component and a microporous catalyst component comprising 8-MR pore openings, wherein the microporous catalyst component is derived from a natural mineral and the product stream comprises a combined $C_2$ and $C_3$ selectivity greater than 40 carbon mol%.

Additional features and advantages will be set forth in the detailed description which follows, and in part will be readily apparent to those skilled in the art from that description or recognized by practicing the embodiments described herein, including the detailed description which follows and the claims.

It is to be understood that both the foregoing general description and the following detailed description describe various embodiments and are intended to provide an overview or framework for understanding the nature and character of the claimed subject matter.

### BRIEF DESCRIPTION OF THE DRAWINGS

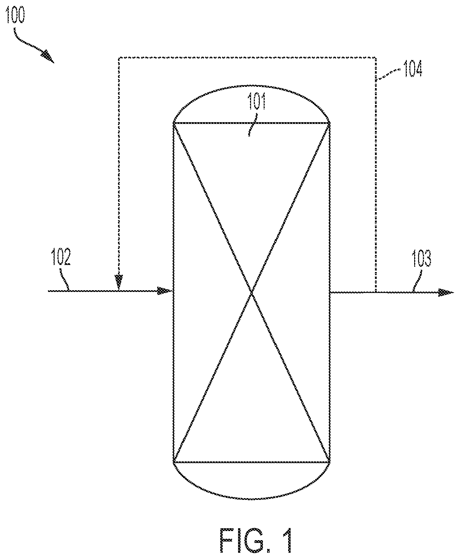

FIG. 1 is a schematic view illustrating two streams being introduced to a reactor and one resulting product stream exiting the reactor in accordance with one or more embodiments of the present disclosure.

### DETAILED DESCRIPTION

Reference will now be made in detail to embodiments of processes utilizing syngas to prepare $C_2$ to $C_3$ hydrocarbons the product stream comprises a combined $C_2$ and $C_3$ selectivity greater than 40 carbon mol% (C mol%) by a hybrid catalyst comprising a metal oxide catalyst component and a microporous catalyst component derived from a natural mineral comprising 8-MR pore openings. As used herein, combined $C_2$ and $C_3$ selectivity is defined as the sum

percentage of $C_2$ and $C_3$ hydrocarbons carbon mole selectivity in the product. As used herein, it is noted that "synthesis gas" and "syngas" are utilized herein to represent a mixture comprising primarily hydrogen, carbon monoxide, and very often some carbon dioxide. Additionally, it is noted that "natural mineral" is utilized herein to refer to a solid inorganic substance of natural occurrence that is not a synthetic material.

As used herein, it is noted that "synthesis gas" and "syngas" are utilized herein to represent a mixture comprising primarily hydrogen, carbon monoxide, carbon dioxide and very often some inerts.

The use of microporous catalyst components when converting feed streams comprising carbon to desired products, such as, for example, $C_2$ to $C_3$ hydrocarbons, is known. In some syngas to hydrocarbon processes, it is desirable to achieve a high productivity of the desired $C_2$ to $C_3$ hydrocarbons, while simultaneously achieving a high catalyst activity. However, known microporous catalyst components may be made synthetically, but may be quite costly and require intensive labor and energy to be produced. Specifically, in some synthetic processes, the intention may be to maximize the amount of $C_2$ hydrocarbons formed.

Processes according to embodiments disclosed and described herein address the combined $C_2$ and $C_3$ selectivity in the product and the amount of $C_2$ hydrocarbons formed by using a microporous catalyst component comprising 8-MR pore openings that are formed from a natural mineral. Syngas to hydrocarbon process with increased combined $C_2$ and $C_3$ selectivity in the product according to embodiments will now be described in more detail.

Referring to the embodiment of FIG. 1, a feed stream 102 is fed into a reaction zone 101, the feed stream 102 may comprise $H_2$ gas and carbon monoxide (CO). In one or more embodiments, the feed stream 102 is syngas. In embodiments, the $H_2$ gas is present in the feed stream 102 in an amount of from 10 volume percent (vol.%) to 90 vol.%, based on combined volume of the $H_2$ gas and CO. In embodiments, the $H_2$ gas is present in the feed stream 102 in an amount from 40 vol.% to 80 vol.% or from 60 vol.% to 80 vol.%, based on combined volume of $H_2$ gas and CO. The feed stream 102 is introduced into the reaction zone 101 and contacted with a hybrid catalyst as disclosed and described herein below in the reaction zone 101. As will be described in more detail herein, the hybrid catalyst comprises a mixed metal oxide catalyst component and a microporous catalyst component. After the feed stream 102 is introduced to the reaction zone 101 and contacted with the hybrid catalyst, a product stream 103 is passed out of the reaction zone 101. The product stream 103 may be separated to form a recycle stream 104 that is, according to embodiments, combined with the feed stream 102 or, according to embodiments, may be introduced to the reaction zone 101 (not shown in FIG. 1) to be combined with the feed stream 102.

As disclosed herein above, the feed stream 102 may comprise $H_2$, CO, $CO_2$, or combinations thereof. The feed stream 102 may, according to embodiments, comprise greater than 10.0 vol.% $H_2$. such as from 10.0 vol.% to 90.0 vol.% $H_2$, from 10.0 vol.% to 80.0 vol.% $H_2$, from 10.0 vol.% to 70.0 vol.% $H_2$, from 10.0 vol.% to 60.0 vol.% $H_2$, from 10.0 vol.% to 50.0 vol.% $H_2$, from 10.0 vol.% to 40.0 vol.% $H_2$, or from 10.0 vol.% to 30.0 vol.% $H_2$. In embodiments, the feed stream 102 comprises from 20.0 vol.% to 90.0 vol.% $H_2$, such as from 30.0 vol.% to 90.0 vol.% $H_2$, from 40.0 vol.% to 90.0 vol.% $H_2$, from 50.0 vol.% to 90.0 vol.% $H_2$, or from 80.0 vol.% to 90.0 vol.% $H_2$. In embodiments, the feed stream 102 comprises from 20.0 vol.% to 80.0 vol.% $H_2$, such as from 40.0 vol.% to 80.0 vol.% $H_2$, or from 60.0 vol.% to 80.0 vol.% $H_2$. In embodiments, the feed stream 102 comprises from 45.0 vol.% to 85.0 vol.% $H_2$, such as from 55.0 vol.% to 85.0 vol.% $H_2$ or from 65.0 vol.% to 85.0 vol.% $H_2$.

Without being bound to any particular theory, it is believed that the coking and decoking rates are nearly balanced, which may significantly reduce coking on the hybrid catalyst. This, in turn, may allow the hybrid catalyst to remain in the reaction zone 101 for extended periods of time without the need for regeneration. In embodiments, the hybrid catalyst may remain in the reaction zone 101 without coking for greater than 5 hours, such as greater than 7.5 hours, greater than 10 hours, greater than 12.5 hours, greater than 15 hours, greater than 17.5 hours, greater than 20 hours, greater than 22.5 hours, greater than 25 hours, greater than 27.5 hours, or greater than 30 hours.

As previously described herein, $C_2$ to $C_3$ hydrocarbons may be particularly desirable as they are useful in downstream applications. In embodiments, the product stream 103 may comprise greater than 40 C mol% $C_2$ to $C_3$ hydrocarbons based on the total hydrocarbon fraction in the product stream 103, such as greater than 42 C mol%, greater than 44 C mol%, greater than 46 C mol%, greater than 48 C mol%, greater than 50 C mol%, greater than 52 C mol%, greater than 54 C mol%, greater than 56 C mol%, greater than 58 C mol%, greater than 60 C mol%. In embodiments, the product stream 103 may comprise less than 99 C mol% $C_2$ to $C_3$ hydrocarbons, such as less than 97 C mol%, less than 95 C mol%, less than 90 C mol%, less than 85 C mol%, less than 75 C mol%, or less than 65 C mol%.

The reaction conditions within the reaction zone 101 will now be described. The feed stream 102 may be contacted with the hybrid catalyst in the reaction zone 101 under reaction conditions sufficient to form the product stream 103 comprising $C_2$ to $C_3$ hydrocarbons. The reaction conditions comprise a temperature within reaction zone 101 ranging, according to one or more embodiments, from 300 °C to 500 °C, such as from 380 °C to 450 °C, from 380 °C to 440 °C, from 380 °C to 430 °C, from 380 °C to 420 °C, from 380 °C to 410 °C, from 380 °C to 400 °C, or from 380 °C to 390 °C. In embodiments, the temperature within the reaction zone 101 is from 390 °C to 450 °C, from 400 °C to 450 °C, from 410 °C to 450 °C, from 420 °C to 450 °C, from 430 °C to 450 °C, or from 440 °C to 450 °C. In embodiments, the temperature within the reaction zone 101 is from 380 °C to 450 °C, such as from 390 °C to 440 °C, from 400 °C to 430 °C, or from 410 °C to 420 °C.

The reaction conditions also, in embodiments, include a pressure inside the reaction zone 101 of at least 2000 kilopascals (kPa) (20 bar) , such as at least 2500 kPa (25 bar), at least 3000 kPa (30 bar), at least 3500 kPa (35 bar) , at least 4000 kPa (40 bar), at least 4500 kPa (45 bar), at least 5000 kPa (50 bar), at least 5500 kPa (55 bar), at least 6000 kPa (60 bar), at least 6500 kPa (65 bar), or at least 7000 kPa (70 bar). In embodiments, the reaction conditions include a pressure inside the reaction zone 101 from 2000 kPa (20 bar) to 7000 kPa (70 bar), such as from 2500 kPa (25 bar) to 6500 kPa (65 bar), or from 3000 kPa (30 bar) to 6000 kPa (60 bar), from 3500 kPa (35 bar) to 5500 kPa (55 bar), from 4000 kPa (40 bar) to 5000 kPa (50 bar).

The reaction conditions also, in embodiments, include a gas hourly space velocity (GHSV) (measured as the volume of the feed stream 102 per volume of the catalyst per hour) inside the reaction zone 101 of at least 500 $hr^{-1}$, such as at least 1000 $hr^{-1}$, such as at least 1200 $hr^{-1}$, such as at least 1800 $hr^{-1}$, such as at least 2400 $hr^{-1}$, such as at least 3000 $hr^{-1}$, such as at least 3600 $hr^{-1}$, such as at least 4200 $hr^{-1}$, such as at least 4800 $hr^{-1}$, such as at least 5400 $hr^{-1}$, such as at least 6000 $hr^{-1}$, such as at least 6600 $hr^{-1}$, or such as at least 7200 $hr^{-1}$. In embodiments, the reaction conditions also include a GHSV inside the reaction zone 101 of less than 15000 $hr^{-1}$, such as less than 14600 $hr^{-1}$, such as less than 14000 $hr^{-1}$, such as less than 13400 $hr^{-1}$, or such as less than 12800 $hr^{-1}$.

The hybrid catalyst used in the above-disclosed processes will now be described. As previously described, the hybrid catalyst systems comprise a metal oxide catalyst component, which converts the feed stream to oxygenated hydrocarbons, and a microporous catalyst component, which converts the oxygenated hydrocarbons to hydrocarbons.

In one or more embodiments, the mixed metal oxide catalyst component may be a bulk catalyst or a supported catalyst and may be made by any suitable method, such as co-precipitation, impregnation, or the like. In embodiments, the mixed metal oxide catalyst component may comprise gallium, lanthanum, or combinations thereof. In embodiments, the mixed metal oxide catalyst component may comprise zirconia. In embodiments, the mixed metal oxide catalyst component may comprise gallium, lanthanum, or combinations thereof supported on zirconia. Additional mixed metal oxide catalyst components are contemplated depending on the product slate determined by the microporous catalyst component. It should be understood that any metal in the mixed metal oxide component mixture can be present in a variety of oxidation states. It should also be understood that the designation of a specific oxide (e.g. $Ga_2O_3$), does not necessarily preclude the presence of an additional or different oxide of the given metal(s).

In embodiments, the mixed metal oxide catalyst component may be reduced within the reactor prior to exposure to the feed stream 102 by exposing the mixed metal oxide catalyst component to conventional reducing gases. In one or more embodiments, the mixed metal oxide catalyst component may be reduced within the reactor upon exposure to reducing gases in the feed stream 102 such as $H_2$ and CO.

The hybrid catalyst, according to embodiments, comprises a mixed metal oxide catalyst component in admixture with a microporous catalyst component that may be selected from molecular sieves having 8-MR pore access where the microporous catalyst component is derived from a natural mineral. The natural mineral may comprise Chabazite, Erionite, or Levyne. Chabazite is a tectosilicate mineral of the zeolite group and may include Chabazite-Ca, Chabazite-K, Chabazite-Na, and Chabazite-Sr. Erionite is a fibrous mineral of the zeolite group. Levyne is a hydrated silicate mineral of the zeolite group and may include Levyne-Na, Levyne-Ca, or Levyne-Cs. Other natural occurring minerals with 8-MR pore access may also be used as source for microporous catalyst component including, but not limited to, Clinoptilolite or Heulandite (both framework type HEU), Phillipsite (framework type PHI), Stilbite (framework type STI), and Natrolite (framework type NAT). These minerals may be produced at commercial scale according to 2016 Minerals Yearbook (U.S. Department of the Interior, August 2018) and IHS Chemical Economic Handbook Report "Zeolites" (16 December, 16 2016).

Microporous catalyst components derived from natural minerals may be preferable over synthetic microporous catalyst components. Natural minerals may be cheap and readily obtainable as raw materials, as compared to synthetic microporous catalyst components. The cost and availability of natural minerals, which may be processed to be microporous catalyst components, results in microporous catalyst components derived from natural minerals being an attractive alternative to synthetic microporous catalyst components. Additionally, as further demonstrated in the Examples, it has been discovered that natural minerals may be substituted for synthetic microporous catalyst components and perform as desired.

The natural mineral may be processed to produce the microporous catalyst component. For example, the natural mineral may be ion exchanged with an aqueous solution of ammonium salts. In another embodiment, the microporous catalyst component may be steamed. Further, the natural mineral does not require synthesis using organic structure-directing agents (SDAs) as is required with a synthetic microporous catalyst component.

In embodiments, the microporous catalyst component may be selected from the CHA, ERI, LEV framework types, and combinations thereof, the framework types corresponding to the naming convention of the International Zeolite Association. In embodiments, the microporous catalyst component may comprise one or more of silica-aluminate or silicoaluminophosphate. It should be understood that in embodiments, both aluminosilicate, silicaaluminate, and silicoaluminophosphate frameworks may be used. In embodiments, the microporous catalyst component may be H-ERI having an Erionite (ERI) framework type.

According to embodiments, the metal component may comprise from 0.1 wt.% to 10.0 wt.% of the mixed metal oxide catalyst component. For example, the metal component may comprise from 0.1 wt.% to 9.0 wt.% of the mixed metal oxide catalyst component, such as from 0.1 wt.% to 1.0 wt.%, from 0.1 wt.% to 2.0 wt.%, from 0.1 wt.% to 3.0 wt.%, from 0.1 wt.% to 4.0 wt.%, from 0.1 wt.% to 5.0 wt.%, from 0.1 wt.% to 6.0 wt.%, from 0.1 wt.% to 7.0 wt.%, from 0.1 wt.% to 8.0 wt.%, from 0.5 wt.% to 1.0 wt.%, from 0.5 wt.% to 2.0 wt.%, from 0.5 wt.% to 3.0 wt.%, from 0.5 wt.% to 4.0 wt.%, from 0.5 wt.% to 5.0 wt.%, from 0.5 wt.% to 6.0 wt.%, from 0.5 wt.% to 7.0 wt.%, from 0.5 wt.% to 8.0 wt.%, from 0.5 wt.% to 9.0 wt.%, from 0.5 wt.% to 10.0 wt.%, from 1.0 wt.% to 2.0 wt.%, from 1.0 wt.% to 3.0 wt.%, from 1.0 wt.% to 4.0 wt.%, from 1.0 wt.% to 5.0 wt.%, from 1.0 wt.% to 6.0 wt.%, from 1.0 wt.% to 7.0 wt.%, from 1.0 wt.% to 8.0 wt.%, from 1.0 wt.% to 9.0 wt.%, or form 1.0 wt.% to 10.0 wt.%. In embodiments, the metal component may comprise from 2.0 wt.% to 10.0 wt.% of the mixed metal oxide catalyst component, such as from 2.0 wt.% to 3.0 wt.%, from 2.0 wt.% to 4.0 wt.%, from 2.0 wt.% to 5.0 wt.%, from 2.0 wt.% to 6.0 wt.%, from 2.0 wt.% to 7.0 wt.%, from 2.0 wt.% to 8.0 wt.%, from 2.0 wt.% to 9.0 wt.%, from 3.0 wt.% to 4.0 wt.%, from 3.0 wt.% to 5.0 wt.%, from 3.0 wt.% to 6.0 wt.%, from 3.0 wt.% to 7.0 wt.%, from 3.0 wt.% to 8.0 wt.%, from 3.0 wt.% to 9.0 wt.%, or form 3.0 wt.% to 10.0 wt.%. In one or more embodiments, the metal component may comprise from 4.0 wt.% to 10.0 wt.% of the mixed metal oxide catalyst component, such as from 4.0 wt.% to 5.0 wt.%, from 4.0 wt.% to 6.0 wt.%, from 4.0 wt.% to 7.0 wt.%, from 4.0 wt.% to 8.0 wt.%, from 4.0 wt.% to 9.0 wt.%, from 5.0 wt.% to 6.0 wt.%, from 5.0 wt.% to 7.0 wt.%, from 5.0 wt.% to 8.0 wt.%, from 5.0 wt.% to 9.0 wt.%, or from 5.0 wt.% to 10.0 wt.%.

According to embodiments, the microporous catalyst component may comprise a $SiO_2/Al_2O_3$ molar ratio less than or equal to 50.0, such as less than or equal to 48.0, less than or equal to 46.0, less than or equal to 44.0, less than or equal to 42.0, less than or equal to 40.0, less than or equal to 38.0, less than or equal to 36.0, less than or equal to 34.0, less than or equal to 32.0, less than or equal to 30.0, less than or equal to 28.0, less than or equal to 26.0, less than or equal to 24.0, less than or equal to 22.0, less than or equal to 20.0, less than or equal to 18.0, less than or equal to 16.0, less than or equal to 14.0, less than or equal to 12.0, or less than or equal to 10.0. In embodiments, the microporous catalyst component may comprise a $SiO_2/Al_2O_3$ molar ratio greater than or equal to 1.0, such as greater than or equal to 1.5, greater than or equal to 2.0, greater than or equal to 2.5, greater than or equal to 3.0, greater than or equal to 3.5, greater than or equal to 4.0, or greater than or equal to 4.5.

Examples of these may include, but are not necessarily limited to ERI embodiments and LEV embodiments. Combinations of microporous catalyst components having any of the above framework types may also be employed. It should be understood that the microporous catalyst component may have a different membered ring pore opening depending on the desired product. For instance, microporous catalyst component having 8-MR to 12-MR pore openings could be used depending on the desired product. However, to produce $C_2$ to $C_3$ hydrocarbons, a microporous catalyst component having 8-MR pore openings is used in embodiments.

Without being bound to any particular theory, it is believed that the pore access size (Atlas of zeolite framework types, 6th edition, Elsevier, pg. 381-86, 2007) and cage defining ring size (ACS Catalysis, 2019, Vol. 9, pg. 6017) may contribute to a desired hydrocarbon product. The pore access size may be important to narrow the product distribution. Additionally, the cage defining ring size may be important for enhancing the $C_2$ fraction in the product distribution. The cage defining ring size may be equivalent to the longest axis of an ellipsoid that may fit into the cage of the microporous catalyst component. In embodiments, where the microporous catalyst component does not comprise a cage, the pore access and the largest channel dimension of the microporous catalyst component may contribute to the desired hydrocarbon product. Together, the pore access size and the cage defining ring size may be tailored to target a hydrocarbon product with a specific product distribution. This theory may be supported by "Cage-Defining Ring: A Molecular Sieve Structural Indicator for Light Olefin Product Distribution from the Methanol-to-Olefins Reaction," John Hun Kang, Faisal H. Alshafei, Stacey I. Zones, & Mark E. Davis, ACS Catalysis 2019 9(7), 6012-6019 (April 11, 2019).

As further demonstrated in the examples below, the microporous catalyst component, prior to combination with the mixed metal oxide catalyst component, may be ion exchanged. Without being bound to any particular theory, the ion exchange may alter the properties of the microporous catalyst component (and therefore, the overall hybrid catalyst) by increasing the acidity of the microporous catalyst component by replacing metal atoms, such as, but not limited to, potassium or sodium, with hydrogen. Additionally, the microporous catalyst component can be steamed or de-aluminated, by methods known to one skilled in the art. As the acidity of the microporous catalyst component is decreased, this may alter the chemistry of the hybrid catalyst to tailor the process to form a desirable product.

The mixed metal oxide catalyst component and the microporous catalyst component of the hybrid catalyst may be mixed together by any suitable means, such as, for example, by physical mixing-such as shaking, stirring, or other agitation. In embodiments, the mixed metal oxide catalyst component and the microporous catalyst component may be present as a single formulated catalyst. The mixed metal oxide catalyst component and the microporous catalyst component may be present in the reaction zone 101, typically as a hybrid catalyst in a catalyst bed, in a weight/weight (wt./wt.) ratio (mixed metal oxide catalyst component:microporous catalyst component) ranging from 0.1:1 to 10:1, such as from 0.5:1 to 9:1.

While the hybrid catalyst described herein may be applicable to processes other than synthesis gas to hydrocarbons type systems, it has been discovered that hybrid catalysts may not be directly transferable between various processes. That is, a catalyst may be an effective catalyst for one process, but may show poor performance in another process. For example, a catalyst demonstrating satisfactory performance in a methanol-to-olefins process may not be directly transferable to a synthesis gas to hydrocarbon process, where the same catalyst may not exhibit the same satisfactory performance. Further, the disclosures and teachings about product distributions in other processes, such as in a methanol-to-olefins (MTO) processes, may not be transferable to synthesis gas to hydrocarbons type systems. For example, a hybrid catalyst comprising SSZ-13 with CHA topology may not demonstrate the same product distributions in different systems, such as MTO, that it provides in synthesis gas to hydrocarbons type systems.

## EXAMPLES

Embodiments will be further clarified by the following examples.

*Example 1: Hybrid Catalyst with H-ERI Microporous Catalyst Component*

In Example 1, a microporous catalyst component was prepared from Erionite. The Erionite, which was sourced from Minerals Research, P.O. Box 591, Clarkson, NY 14430, was collected from Eastgate, Nevada. Roughly 10 g of Erionite 25220 was placed in a flint glass jar with a stir bar. Then, 100 mL of 0.1 M ammonium acetate was added. A series of three ion exchanges were performed. After each ion exchange, the solids were recovered by filtration, rinsed, and then re-suspended. In the first exchange, the Erionite and ammonium acetate were stirred at ambient temperature for eighteen hours. In the second exchange (after filtration, rinse, and re-suspension), the Erionite and ammonium acetate were stirred at ambient temperature for five hours. In the third exchange (after filtration, rinse, and re-suspension), the Erionite and ammonium acetate were again stirred at ambient temperature for eighteen hours. After the third and final exchange, the final products were collected by filtration, rinsed, and then dried at 90 °C. Following drying, the product was calcined in air, by ramping the temperature up to 550 °C at 2.5 °C per minute. The temperature was then held at 550 °C for four hours. The calcined powder was then compacted and sized to 60-100 mesh size to form the H-ERI microporous catalyst component.

To prepare the mixed metal oxide catalyst component, an impregnation solution of 4.40 mL of gallium stock solution (gallium (III) nitrate hydrate with C = 2.0 M in deionized water), 1.76 mL of lanthanum stock solution (lanthanum (III) nitrate hexahydrate with C = 1.5M in deionized water), and 2.86 mL of deionized water was mixed. Then, 20 g of 60-80 mesh size ZrOz support (NORPRO© SZ31164) with a Brunauer-Emmett-Teller of 100 m$^2$/g, a pore volume of 0.41 mL/g (measured by deionized water), and a 100% monoclinic phase (measured by XRD) was placed in a glass vial. The impregnation solution was then added dropwise to the ZrOz support while shaking. Once the impregnation solution had been combined with the $ZrO_2$ support, the mixture was dried at 120 °C in static air. Then, the product was calcined by ramping the temperature to up to 120 °C at 2.0 °C per minute. After the temperature was held at 120 °C for two hours, the temperature was again ramped up, this time to 550 °C at 3.0 °C per minute. After the temperature was held at 550 °C for four hours, the product was cooled down to room temperature over a period of two hours. After calcination, the catalyst was re-sieved to 60-100 mesh size to remove any fine particles. As determined by X-ray fluorescence, the mixed metal oxide catalyst component had a composition of 3.1 wt.% Ga, 1.5 wt.% La, 67.3 wt.% Zr, 2.4 wt.% Hf, and 25.7 wt.% O.

To prepare the hybrid catalyst, 100 mg of the H-ERI microporous catalyst component was mixed with 50 mg of the mixed metal oxide catalyst component were and shaken for thirty seconds.

*Example 2: Hybrid Catalyst with H-ERI Microporous Catalyst Component*

In Example 2, the microporous catalyst component and the mixed metal oxide catalyst component were prepared using the same procedure as Example 1. When preparing the hybrid catalyst, 112.5 mg of the H-ERI microporous catalyst component was mixed with 37.5 mg of the mixed metal oxide catalyst component were and shaken for thirty seconds.

*Example 3: Hybrid Catalyst with LZ-220 Microporous Catalyst Component*

To prepare the microporous catalyst component, the H-ERI microporous catalyst component of Example 1 was further treated to produce the LZ-220 microporous catalyst component. LZ-220 is further described in US Patent No. 4,503,023. First, an ammonium hexafluorosilicate stock solution was prepared by dissolving 8.0 g $(NH_4)_2SiF_6$ in 250 mL of high purity water. Then 5.0 g of ion-exchanged $NH_4$-ERI (that is, the microporous catalyst component of Example 1) was suspended in 100 mL of high purity water in a flint glass jar with a stir bar, which was immersed in a water bath while stirring vigorously. While the $NH_4$-ERI solution was heated to 80 °C, 50 mL of the ammonium hexafluorosilicate solution was added to the NH$_4$-ERI solution. Then, the resulting mixture was heated to 90 °C and held at 90 °C for three hours. After the solution was cooled for one hour, solids were collected by filtration and rinsed. The final solids were then dried at 90 °C. Then, the as-prepared microporous catalyst component was dried and calcined on air by ramping the temperature up to 550 °C at 2.5 °C per minute. The temperature was then held at 550 °C for four hours. The calcined powder was then compacted and sized to 60-100 mesh size to form the LZ-220 microporous catalyst component.

The mixed metal oxide catalyst component was prepared in the same manner as Example 1. Finally, 100 mg of the LZ-220 microporous catalyst component was mixed with 50 mg of the mixed metal oxide catalyst component were and shaken for thirty seconds.

*Example 4: Hybrid Catalyst with LZ-220 Microporous Catalyst Component*

In Example 4, the microporous catalyst component and the mixed metal oxide catalyst component were prepared using the same procedure as Example 3. When preparing the hybrid catalyst, 112.5 mg of the LZ-220 microporous catalyst component was mixed with 37.5 mg of the mixed metal oxide catalyst component were and shaken for thirty seconds.

*Example 5: Hybrid Catalyst with H-CHA Microporous Catalyst Component*

In Example 5, to prepare the microporous catalyst component, 3 g of natural Chabazite were placed in a 500 mL beaker. Then, 300 mL of 1M NH$_4$NO$_3$ solution in deionized water was added to the beaker. The slurry was vigorously stirred at 70 °C by a mechanical stirrer for two hours at 400 rpm. The resulting mixture was then filtered through a paper filter on a plastic funnel. The filtered solids were washed with 1 L of deionized water. The previously described ion exchange was repeated two more times. The as-prepared solid was allowed to dry at room temperature overnight. Then, the temperature was ramped up to 120 °C at 2 °C per minute and kept at 120 °C for two hours. Then, the as-prepared solid was calcined on air by ramping the temperature up to 550 °C at 3 °C per minute and kept at 550 °C for four hours. Finally, the calcined powder was then compacted and sized to 60-100 mesh size to form the H-CHA microporous catalyst component.

To prepare the mixed metal oxide catalyst component, an impregnation solution of 4.0 mL of gallium stock solution (gallium (III) nitrate hydrate with C = 2.0 M in deionized water), 3.852 mL of lanthanum stock solution (lanthanum (III) nitrate hexahydrate with C = 0.623 M in deionized water), and 0.148 mL of deionized water was mixed. Then, 15 g of 60-80 mesh size ZrOz support (NORPRO© SZ31164) with a Brunauer-Emmett-Teller of 100 m$^2$/g, a pore volume of 0.41 mL/g (measured by deionized water), and a 100% monoclinic phase (measured by XRD) was placed in a glass vial. About 6.0 mL of the impregnation solution was then added dropwise to the ZrOz support while shaking. Once the impregnation solution had been combined with the ZrO$_2$ support, the mixture was dried at 120 °C in static air. Then, the product was calcined by ramping the temperature to up to 120 °C at 2.0 °C per minute. After the temperature was held at 120 °C for two hours, the temperature was again ramped up, this time to 550 °C at 3.0 °C per minute. After the temperature was held at 550 °C for four hours, the product was cooled down to room temperature over a period of two hours. After calcination, the catalyst was re-sieved to 60-100 mesh size to remove any fine particles. As determined by X-ray fluorescence, the mixed metal oxide catalyst component had a composition of 1.84 wt.% Ga, 1.36 wt.% La, 68.5 wt.% Zr, 2.25 wt.% Hf, and 26.05 wt.% O.

The hybrid catalyst was then prepared by combining 145.9 mg of the H-CHA microporous catalyst component with 216.8 mg of the mixed metal oxide catalyst component were and shaken for thirty seconds.

*Example 6: Hybrid Catalyst with H-CHA Microporous Catalyst Component*

In Example 6, the microporous catalyst component and the mixed metal oxide catalyst component were prepared using the same procedure as Example 5. When preparing the hybrid catalyst, 150.8 mg of the H-CHA microporous catalyst component was mixed with 98.1 mg of the mixed metal oxide catalyst component were and shaken for thirty seconds.

*Example 7: Hybrid Catalyst with H-CHA Microporous Catalyst Component*

In Example 7, the microporous catalyst component was prepared using the same procedure as Example 5.

To prepare the mixed metal oxide catalyst component, an impregnation solution of 2.87 mL of indium stock solution (indium (III) nitrate hydrate with C = 1.553 M in deionized water), 2.118 mL of lanthanum stock solution (lanthanum (III) nitrate hexahydrate with C = 0.623 M in deionized water), and 3.011 mL of deionized water was mixed. Then, 15 g of 60-80 mesh size ZrO$_2$ support (NORPRO© SZ31164) with a Brunauer-Emmett-Teller of 100 m$^2$/g, a pore volume of 0.41 mL/g (measured by deionized water), and a 100% monoclinic phase (measured by XRD) was placed in a glass vial. About 6.0 mL of the impregnation solution was then added dropwise to the ZrOz support while shaking. Once the impregnation solution had been combined with the $ZrO_2$ support, the mixture was dried at 120 °C in static air. Then, the product was calcined by ramping the temperature to up to 120 °C at 2.0 °C per minute. After the temperature was held at 120 °C for two hours, the temperature was again ramped up, this time to 550 °C at 3.0 °C per minute. After the temperature was held at 550 °C for four hours, the product was cooled down to room temperature over a period of two hours. After calcination, the catalyst was re-sieved to 60-100 mesh size to remove any fine particles. As determined by X-ray fluorescence, the mixed metal oxide catalyst component had a composition of 2.4 wt.% Ga, 0.73 wt.% La, 68.8 wt.% Zr, 2.3 wt.% Hf, and 25.77 wt.% O.

The hybrid catalyst was then prepared by combining 151.4 mg of the H-CHA microporous catalyst component with 100.7 mg of the mixed metal oxide catalyst component were and shaken for thirty seconds.

*Example 8: Hybrid Catalyst with H-CHA Microporous Catalyst Component*

In Example 8, the microporous catalyst component was prepared using the same procedure as Example 5.

The mixed metal oxide catalyst component was commercially available Cu-based methanol synthesis catalyst HiFuel™ R120 that has a Cu content of 51 wt.%, a Zn content of 20 wt.%, and an Al content of 5 wt.%. The catalyst was crushed and re-sieved to 60-100 mesh size.

The hybrid catalyst was then prepared by combining 150.4 mg of the H-CHA microporous catalyst component with 102.1 mg of the mixed metal oxide catalyst component were and shaken for thirty seconds.

*Example 9: Hybrid Catalyst with H-CHA Microporous Catalyst Component*

In Example 9, the microporous catalyst component was prepared using the same procedure as Example 5.

To prepare the mixed metal oxide catalyst component, an impregnation solution of 0.983 mL of zinc stock solution (zinc (II) nitrate hydrate with C = 2.5 M in deionized water), and 1.867 mL of deionized water was mixed. Then, 1 g of 60-80 mesh size $TiO_2$ support (NORPRO© ST61120) with a Brunauer-Emmett-Teller of 140 $m^2$/g, a pore volume of 0.57 mL/g (measured by deionized water), and a 100% anatase phase (measured by XRD) was placed in a glass vial. Then, 0.57 mL of the impregnation solution was then added dropwise to the $TiO_2$ support while shaking. Once the impregnation solution had been combined with the $TiO_2$ support, the mixture was dried at 120 °C in static air. Then, the product was calcined by ramping the temperature to up to 120 °C at 2.0 °C per minute. After the temperature was held at 120 °C for two hours, the temperature was again ramped up, this time to 400 °C at 3.0 °C per minute. After the temperature was held at 400 °C for four hours, the product was cooled down to room temperature over a period of two hours. After calcination, the catalyst was re-sieved to 60-100 mesh size to remove any fine particles. As determined by X-ray fluorescence, the mixed metal oxide catalyst component had a composition of 3.18 wt.% Zn, 56.9 wt.% Ti, 0.14 wt.% S, and the balance oxygen O.

The hybrid catalyst was then prepared by combining 155.2 mg of the H-CHA microporous catalyst component with 100.2 mg of the mixed metal oxide catalyst component were and shaken for thirty seconds.

*Example 10: Hybrid Catalyst with H-CHA Microporous Catalyst Component*

In Example 10, the microporous catalyst component was prepared using the same procedure as Example 5.

To prepare the mixed metal oxide catalyst component, an impregnation solution of 4.754 mL of zinc stock solution (zinc (II) nitrate hydrate with C = 1.473 M in deionized water), and 3.246 mL of deionized water was mixed. Then, 10 g of 60-80 mesh size ZrOz support (NORPRO© SZ31164) with a Brunauer-Emmett-Teller of 100 $m^2$/g, a pore volume of 0.41 mL/g (measured by deionized water), and a 100% monoclinic phase (measured by XRD) was placed in a glass vial. Then, about 4.0 mL of the impregnation solution was then added dropwise to the $ZrO_2$ support while shaking. Once the impregnation solution had been combined with the $ZrO_2$ support, the mixture was dried at 120 °C in static air. Then, the product was calcined by ramping the temperature to up to 120 °C at 2.0 °C per minute. After the temperature was held at 120 °C for two hours, the temperature was again ramped up, this time to 550 °C at 3.0 °C per minute. After the temperature was held at 550 °C for four hours, the product was cooled down to room temperature over a period of two hours. After calcination, the catalyst was re-sieved to 60-100 mesh size to remove any fine particles. As determined by X-ray fluorescence, the mixed metal oxide catalyst component had a composition of 2.18 wt.% Zn, 69.6 wt.% Zr, 2.35 wt.% Hf, and the balance oxygen O.

The hybrid catalyst was then prepared by combining 147.7 mg of the H-CHA microporous catalyst component with 101.9 mg of the mixed metal oxide catalyst component were and shaken for thirty seconds.

*Example 11: Hybrid Catalyst with H-CHA Microporous Catalyst Component*

In Example 11, the microporous catalyst component was prepared using the same procedure as Example 5.

The mixed metal oxide catalyst component was commercially available $ZrO_2$ mixed metal oxide (NORPRO© SZ31108) with a Brunauer-Emmett-Teller of 70 $m^2/g$ and a 100% monoclinic phase (measured by XRD). The catalyst was crushed and re-sieved to 60-100 mesh size.

The hybrid catalyst was then prepared by combining 151.1 mg of the H-CHA microporous catalyst component with 101.2 mg of the mixed metal oxide catalyst component were and shaken for thirty seconds.

*Comparative Example 1: Hybrid Catalyst with SAPO-34 Microporous Catalyst Component*

To prepare the SAPO-34 microporous catalyst component, the microporous catalyst component was synthesized according to the procedure of U.S. Patent 4,440,871 A, was used. The as-formed microporous catalyst component was then calcined on air by ramping the temperature from 25 °C to 600 °C at 5 °C per minute. The temperature was kept at 600 °C for four hours before the temperature was reduced to 25 °C over a period of four hours. The calcined powder was then compacted and sized to 60-100 mesh size to form the SAPO-34 microporous catalyst component.

In Comparative Example 1, the mixed metal oxide catalyst component and the preparation of the hybrid catalyst are the same as Example 1. The hybrid catalyst was formed by combining 50 mg of the microporous catalyst component with 100 mg of the mixed metal oxide catalyst component and shaking for 30 seconds.

*Comparative Example 2: Hybrid Catalyst with SAPO-34 Microporous Catalyst Component*

In Comparative Example 2, the hybrid catalyst was formed using the same microporous catalyst component and same the mixed metal oxide catalyst component of Comparative Example 2. The hybrid catalyst was formed by combining 112.5 mg of the microporous catalyst component with 37.5 mg of the mixed metal oxide catalyst component and shaking for 30 seconds.

*Comparative Example 3: Hybrid Catalyst with SSZ-13 Microporous Catalyst Component*

In Comparative Example 3, the microporous catalyst component was SSZ-13. The microporous catalyst component was prepared following the synthesis described in Chemical Communications 2016, 42, 3227. More specifically, 41.2 g high purity water was added to a plastic beaker with a stir bar. Then 13.6 g of the 25 wt.% template hydroxide solution was added to the plastic beaker followed by 0.96 g of the 50 wt.% NaOH solution. This mixture was stirred. Then, 0.32 g $Al(OH)_3$ was added. Again, the mixture was stirred for thirty minutes or until the $Al(OH)_3$ was dissolved. Finally, 16 g Ludox AS-30 was rapidly added to the mixture to form a slurry. The slurry was stirred at ambient conditions overnight. Then, the slurry was poured into two 45 mL Teflon-lined Parr digestion reactors and held at 160 °C for seven days. The solids were then collected by filtration, rinsed on the filter, and dried at 90 °C.In Comparative Example 3, the same mixed metal oxide catalyst component as Example 5 was used. The hybrid catalyst was formed by combining 100.8 mg of the microporous catalyst component with 148.5 mg of the mixed metal oxide catalyst component and shaking for 30 seconds.

*Comparative Example 4: Hybrid Catalyst with CHA Microporous Catalyst Component*

The microporous catalyst component of Comparative Example 4 was a natural mineral Chabazite that was pelletized and sized to 60-80 mesh size.

In Comparative Example 4, the same mixed metal oxide catalyst component as Example 5 was used. The hybrid catalyst was formed by combining 117.1 mg of the microporous catalyst component with 214.9 mg of the mixed metal oxide catalyst component and shaking for 30 seconds.

*Comparative Example 5: Hybrid Catalyst with H-CHA Microporous Catalyst Component*

In Comparative Example 5, the microporous catalyst component was the same of that formed in Example 5. For the mixed metal oxide catalyst component, a commercially available $TiO_2$ support (NORPOR© ST61120) with a Brunauer-Emmett-Teller of 140 $m^2/g$, a pore volume of 0.57 mL/g (measured by deionized water), and a 100% anatase phase (measured by XRD) was used. The support was crushed and re-sieved to 60-80 mesh size.

In Comparative Example 5, the hybrid catalyst was formed by combining 151.1 mg of the microporous catalyst component with 101.2 mg of the mixed metal oxide catalyst component and shaking for 30 seconds.

Compositions of Microporous Catalyst Components of Examples 1-11 and Comparative Examples 1-5

The compositions of the microporous catalyst components of Examples 1-11 and Comparative Examples 1-5 are shown in Table 1.

Table 1. Compositions of Examples 1-11 and Comparative Examples 1-5

| | Na wt.% Percent | Mg wt.% Percent | Al wt.% Percent | Si wt.% Percent | s wt.% Percent | P wt.% Percent | K wt.% Percent | Ca wt.% Percent | Ti wt.% Percent | Fe wt.% Percent | Zr wt.% Percent | $SiO_2/Al_2O_3$ (mol/mol) |
|---|---|---|---|---|---|---|---|---|---|---|---|---|
| ERI | 7.0 | 0.3 | 8.5 | 29.6 | 0.9 | 0.0 | 0.3 | 3.9 | 0.3 | 1.8 | | 6.8 |
| CHA (Comp. Ex. 4) | 0.6 | 0.9 | 8.6 | 32.4 | 0.272 | 0.014 | 2.32 | 3.84 | 0.18 | 1.9 | 0.06 | 7.3 |
| H-ERI (Ex. 1 and 2) | 0.3 | 0.2 | 9.4 | 34.1 | 0.3 | 0.0 | 0.0 | 4.3 | 0.1 | 1.5 | | 7.0 |
| LZ-220 (Ex. 3 and 4) | 0.2 | 0.2 | 8.4 | 353 | 0.3 | 0.0 | 0.0 | 3.7 | 0.1 | 1.5 | | 8.1 |
| H-CHA (Ex. 5-11; Comp. Ex. 5) | 0.0 | 0.7 | 9.3 | 35.7 | 0.000 | 0.014 | 0.44 | 1.09 | 0.16 | 1.7 | | 7.4 |
| SAPO-34 (Comp. Ex 1 and 2) | | | 22.3 | 4.05 | | 21.1 | | | | | 0.0 | |
| SSZ-13 (Comp. Ex. 3) | | | 4.6 | 42.6 | | | | | | 0.0352 | | 17.8 |

Catalytic Test of Example 1-11 and Comparative Examples 1-5

Catalytic tests were performed in a tubular stainless steel reactor with an inner diameter of 3 mm or a quartz fixed-bed reactor with an inner diameter of 2 mm. The bottom of the stainless steel reactor features a metal frit to hold the catalyst bed. The bottom of the quartz reactor is filled with quartz chips with wool on top to hold the catalyst bed. In a catalytic test, the hybrid catalyst is loaded to the catalyst and the following procedure is followed: nitrogen is flowed to the reactor as the temperature is raised from 25 °C to the reaction temperature, increasing at 5 °C per minute. Similarly, the pressure is increased from ambient conditions to the reaction pressure. Then, the nitrogen flow is replaced with a flow of synthesis gas. The synthesis gas flow is continued for one hour for flushing prior to the gas chromatography analysis beginning. After a set run time duration, the syngas flow is replaced with nitrogen flow as the reactor returns from reaction temperature and pressure to ambient temperature and pressure.

Two different reaction conditions were utilized in the catalytic test of Examples 1-11 and Comparative Examples 1-5, which are shown in Table 2.

Table 2. Reaction Conditions

|  | $H_2$ (vol. %) | CO (vol. %) | He (vol. %) | GHSV ($h^{-1}$) | T (°C) | P (MPa) ((bara)) | Time on Stream (h) |
|---|---|---|---|---|---|---|---|
| Condition 1 | 67.5 | 22.5 | 10 | 4500 | 420 | 4 (40) | 30-60 |
| Condition 2 | 67.5 | 22.5 | 10 | 4800 | 420 | 4 (40) | 10-23 |
| Condition 3 | 67.5 | 22.5 | 10 | 1888 | 430 | 4 (40) | 25-37 |
| Condition 4 | 67.5 | 22.5 | 10 | 1888 | 440 | 4 (40) | 40-70 |
| Condition 5 | 67.5 | 22.5 | 10 | 2153 | 440 | 4 (40) | 40-70 |
| Condition 6 | 67.5 | 22.5 | 10 | 2153 | 450 | 4 (40) | 70-100 |

Finally, the catalytic data for various catalytic tests of Examples 1-11 and Comparative Examples 1-6 is shown in Table 3. Additionally, the selectivity of the products is further detailed in Table 4. Products were analyzed using gas chromatography. Online analysis of components ($N_2$, $H_2$, He, CO, $CO_2$, $C_1$-$C_5$ alkanes, $C_2$-$C_5$ olefins, methanol, and dimethyl ether) was performed periodically to monitor reaction progress. In all experiments, mass balance was 100 $\pm$ 5% based on carbon.

Table 3. Catalytic Data of Examples 1-11 and Comparative Examples 1-5

|  | Condition | Conversion (C mol.%) | Carbon Balance (C mol.%) | $C_2$/$C_3$ Molar Ratio |
|---|---|---|---|---|
| Example 1 | 1 | 47.5 | 98.1 | 1.1 |
| Example 2 | 1 | 41.9 | 99.1 | 1.3 |
| Example 3 | 1 | 42.6 | 97.8 | 1.2 |
| Example 4 | 1 | 42.3 | 98.0 | 1.4 |
| Example 5 | 2 | 52.6 | 992 | 0.8 |
| Example 5 | 3 | 63.7 | 98.4 | 0.7 |
| Example 5 | 4 | 67.4 | 99.3 | 0.6 |
| Example 6 | 5 | 56.1 | 97.4 | 0.2 |
| Example 6 | 6 | 55.5 | 97.9 | 0.2 |
| Example 7 | 5 | 36.6 | 98.7 | 02. |
| Example 7 | 6 | 30.9 | 99.0 | 0.2 |
| Example 8 | 5 | 47.5 | 98.6 | 1.0 |
| Example 8 | 6 | 36.7 | 99.2 | 1.2 |
| Example 9 | 5 | 43.5 | 94.9 | 0.3 |
| Example 10 | 5 | 60.0 | 94.4 | 0.2 |

(continued)

|  | Condition | Conversion (C mol.%) | Carbon Balance (C mol.%) | $C_2/C_3$ Molar Ratio |
|---|---|---|---|---|
| Example 11 | 5 | 21.6 | 96.3 | 0.3 |
|  |  |  |  |  |
| Comparative Example 1 | 1 | 58.1 | 97.9 | 0.3 |
| Comparative Example 2 | 1 | 58.6 | 97.7 | 0.3 |
| Comparative Example 3 | 2 | 71.2 | 100.2 | 0.1 |
| Comparative Example 3 | 3 | 71.9 | 99.6 | 0.1 |
| Comparative Example 3 | 4 | 69.3 | 99.9 | 0.2 |
| Comparative Example 4 | 2 | 9.0 | 99.1 | 1.6 |
| Comparative Example 4 | 3 | 8.6 | 98.3 | 1.7 |
| Comparative Example 4 | 4 | 8.6 | 982 | 1.7 |
| Comparative Example 5 | 5 | 2.9 | 99.6 | 1.4 |

Table 4. Selectivity of Examples 1-11 and Comparative Examples 1-5

|  | Methane | Ethylene | Propylene | Ethane | Propane | Oxygenates* | $CO_2$ |
|---|---|---|---|---|---|---|---|
| Example 1 | 5.1 | 13.4 | 10.0 | 12.8 | 14.2 | 0.5 | 38.0 |
| Example 2 | 5.7 | 13.5 | 9.0 | 13.7 | 13.0 | 0.9 | 40.1 |
| Example 3 | 5.3 | 15.4 | 11.3 | 10.7 | 11.1 | 0.6 | 38.3 |
| Example 4 | 6.1 | 15.8 | 11.0 | 11.5 | 8.6 | 0.7 | 39.3 |
| Example 5 | 9.0 | 2.2 | 6.2 | 18.9 | 19.3 | 0.4 | 40.1 |
| Example 5 | 8.0 | 1.5 | 6.5 | 183 | 23.2 | 0.1 | 37.4 |
| Example 5 | 7.5 | 1.3 | 5.9 | 17.8 | 27.7 | 0.1 | 36.0 |
| Example 6 | 3.6 | 1.1 | 0.0 | 6.6 | 44.6 | 0.0 | 34.7 |
| Example 6 | 4.3 | 1.2 | 0.0 | 6.9 | 44.0 | 0.0 | 35.0 |
| Example 7 | 5.2 | 1.5 | 0.0 | 7.0 | 38.7 | 0.3 | 40.4 |
| Example 7 | 6.0 | 1.8 | 0.0 | 7.0 | 37.4 | 0.3 | 40.7 |
| Example 8 | 5.6 | 0.0 | 0.0 | 24.3 | 23.9 | 0.2 | 39.1 |
| Example 8 | 7.7 | 0.0 | 0.0 | 25.9 | 20.9 | 0.3 | 40.0 |
| Example 9 | 10.7 | 1.3 | 0.0 | 7.0 | 32.8 | 0.0 | 32.7 |
| Example 10 | 4.6 | 0.9 | 0.0 | 6.2 | 41.2 | 0.0 | 33.1 |
| Example 11 | 4.8 | 1.2 | 0.0 | 8.5 | 28.6 | 0.0 | 37.4 |
| Comparative Example 1 | 0.9 | 8.5 | 31.2 | 2.8 | 10.4 | 0.1 | 34.4 |
| Comparative Example 2 | 0.9 | 9.6 | 31.2 | 2.4 | 8.6 | 0.2 | 35.0 |
| Comparative Example 3 | 3.9 | 0.7 | 0.0 | 5.1 | 48.3 | 0.1 | 35.3 |
| Comparative Example 3 | 4.5 | 0.7 | 0.0 | 5.7 | 47.3 | 0.1 | 34.6 |
| Comparative Example 3 | 4.9 | 0.9 | 0.1 | 6.2 | 46.2 | 0.1 | 34.9 |
| Comparative Example 4 | 5.2 | 2.3 | 1.8 | 1.6 | 0.7 | 39.5 | 36.2 |
| Comparative Example 4 | 7.1 | 3.1 | 2.4 | 2.4 | 0.9 | 233 | 38.4 |

(continued)

|  | Methane | Ethylene | Propylene | Ethane | Propane | Oxygenates* | $CO_2$ |
|---|---|---|---|---|---|---|---|
| Comparative Example 4 | 8.8 | 4.1 | 3.1 | 3.4 | 1.3 | 12.1 | 41.8 |
| Comparative Example 5 | 21.5 | 3.8 | 0.0 | 10.8 | 10.1 | 0.0 | 400 |

Table 4. Selectivity of Examples 1-11 and Comparative Examples 1-5 (Continued)

|  | $C_4$ Olefins | $C_5$ Olefins | $C_4$ Paraffins | $C_5$ Paraffins |
|---|---|---|---|---|
| Example 1 | 1.7 | 0.0 | 0.6 | 0.0 |
| Example 2 | 2.1 | 0.0 | 0.6 | 0.0 |
| Example 3 | 2.2 | 0.0 | 0.6 | 0.0 |
| Example 4 | 2.1 | 0.0 | 0.5 | 0.0 |
| Example 5 | 2.1 | 0.0 | 0.8 | 0.0 |
| Example 5 | 2.0 | 0.0 | 0.9 | 0.0 |
| Example 5 | 1.9 | 0.0 | 1.0 | 0.0 |
| Example 6 | 0.1 | 0.0 | 5.1 | 0.0 |
| Example 6 | 0.1 | 0.0 | 5.1 | 0.0 |
| Example 7 | 0.9 | 0.0 | 3.3 | 0.0 |
| Example 7 | 1.1 | 0.0 | 3.5 | 0.0 |
| Example 8 | 0.0 | 0.0 | 4.6 | 0.0 |
| Example 8 | 0.0 | 0.0 | 4.0 | 0.0 |
| Example 9 | 0.2 | 0.0 | 3.6 | 0.0 |
| Example 10 | 0.1 | 0.0 | 4.5 | 0.0 |
| Example 11 | 0.7 | 0.0 | 1.8 | 0.0 |
|  |  |  |  |  |
| Comparative Example 1 | 6.8 | 0.1 | 1.5 | 0.1 |
| Comparative Example 2 | 7.0 | 0.1 | 1.4 | 0.1 |
| Comparative Example 3 | 0.4 | 0.0 | 6.9 | 0.0 |
| Comparative Example 3 | 0.4 | 0.0 | 6.6 | 0.0 |
| Comparative Example 3 | 0.4 | 0.0 | 6.5 | 0.0 |
| Comparative Example 4 | 4.2 | 1.0 | 0.0 | 0.7 |
| Comparative Example 4 | 4.8 | 1.0 | 0.0 | 0.0 |
| Comparative Example 4 | 5.6 | 1.0 | 0.0 | 0.0 |
| Comparative Example 5 | 0.0 | 0.0 | 0.0 | 0.0 |

As can be seen from Tables 3 and 4, hybrid catalysts comprising a microporous catalyst component comprising 8-MR pore openings less than or equal to 0.51 nm (5.1 Å) which are derived from a natural mineral (Examples 1-11) may be capable of converting synthesis gas to hydrocarbons with a high combined $C_2$ and $C_3$ selectivity (such as, greater than 40 C mol%) with a $C_2/C_3$ molar ratio greater than 1.0. Examples 1-4 describe a hybrid catalyst with a microporous catalyst component derived from natural Erionite and Examples 5-11 describe a hybrid catalyst with a microporous catalyst component derived from natural Chabazite. The hybrid catalysts in Examples 5-11 also describe catalysts with different mixed metal oxide catalyst components (such as, Ga-La/$ZrO_2$, In-La/$ZrO_2$, Cu-Zn-Al, Zn/$TiO_2$, Zn/$ZrO_2$ as well as pure $ZrO_2$) and the data in Tables 4 and 5 demonstrates that the natural mineral is effective with various oxides.

Conversely, hybrid catalysts not comprising a microporous catalyst component comprising 8-MR pore openings less than or equal to 0.51 nm (5.1 Å) and are not derived from a natural mineral (Comparative Examples 1-5) may not be capable of converting synthesis gas to hydrocarbons with a high combined $C_2$ and $C_3$ selectivity (such as, greater than 40 C mol%) with a $C_2/C_3$ molar ratio greater than 1.0.

*Calculations for Catalyst Performance for Examples and Comparative Examples*

Carbon monoxide conversion (Xco [C mol%]) is defined as the ratio between molar carbon monoxide flow leaving the reaction zone to carbon monoxide flow entering the reaction zone. The formula for calculating the carbon monoxide conversion is shown below in Equation 1:

$$X_{CO} = \frac{F_{CO,in} - F_{CO,out}}{F_{CO,in}} * 100 \qquad \text{(Equation 1)}$$

where $F_{CO,in}$ is a molar flow of carbon monoxide entering the reaction zone, measured in mol/h, and $F_{CO,out}$ is a molar flow of carbon monoxide leaving the reaction zone, measured in mol/h.

The selectivity [C mol%] of i component is measured as an average of all data points for a time-on-stream specified in Tables 3 and 4. The formula for calculating selectivity of i component is shown below in Equation 2:

$$S_i = \frac{n_i * F_i}{F_{CO,in} - F_{CO,out}} * 100 \qquad \text{(Equation 2)}$$

where $n_i$ is a number of carbon atoms in i component, $F_i$ is a molar flow of i component leaving the reaction zone, measured in mol/h, $F_{CO,in}$ is a molar flow of carbon monoxide entering the reaction zone, measured in mol/h, and $F_{CO,out}$ is a molar flow of carbon monoxide leaving the reaction zone, measured in mol/h.

Carbon balance (CB) [C mol%] is the ratio between the total amount of carbon entering the reaction zone in the form of carbon monoxide and carbon leaving the reaction zone in the form of carbon monoxide and carbon-containing products. The formula for calculating the carbon balance is shown below in Equation 3:

$$CB = \frac{\sum n_i * F_i}{F_{CO,in}} * 100 \qquad \text{(Equation 3)}$$

where $n_i$ is a number of carbon atoms in i component, $F_i$ is a molar flow of i component leaving the reaction zone, measured in mol/h, and $F_{CO,in}$ is a molar flow of carbon monoxide entering the reaction zone, measured in mol/h.

$C_2/C_3$ ratio was calculated using Equation 4, shown below:

$$C_2/C_3 \text{ratio} = \frac{2 * (F_{ethylene} + F_{ethane})}{3 * (F_{propylene} + F_{propane})} * 100 \qquad \text{(Equation 4)}$$

where $F_{propylene}$ and $F_{propane}$ are the molar flows of propylene and propane, respectively, measured in mol/h, and $F_{ethylene}$ and $F_{ethane}$ are the molar flows of ethylene and ethane, respectively, measured in mol/h.

**Claims**

1. A process for preparing $C_2$ to $C_3$ hydrocarbons comprising:

   introducing a feed stream comprising hydrogen gas and a carbon-containing gas selected from the group consisting of carbon monoxide, carbon dioxide, and mixtures thereof into a reaction zone of a reactor; and converting the feed stream into a product stream comprising $C_2$ to $C_3$ hydrocarbons in the reaction zone in the presence of a hybrid catalyst, the hybrid catalyst comprising:

      a mixed metal oxide catalyst component; and
      a microporous catalyst component comprising 8-MR pore openings, wherein:

the microporous catalyst component is derived from a natural mineral; and
the product stream comprises a combined $C_2$ and $C_3$ selectivity greater than 40 C mol% in the product stream.

2. The process of claim 1, wherein the microporous catalyst component comprises a Chabazite, Erionite, or Levyne structure.

3. The process of claim 1, wherein the microporous catalyst component comprises a Heulandite, Phillipsite, Stilbite, or Natrolite structure.

4. The process of any one of claims 1 to 3, wherein the microporous catalyst component comprises a $SiO_2/Al_2O_3$ molar ratio less than or equal to 50.0.

5. The process of any one of claims 1 to 3, wherein the microporous catalyst component comprises a $SiO_2/Al_2O_3$ molar ratio less than or equal to 10.0.

6. The process of any one of claims 1 to 5, wherein the metal component comprises from 0.1 wt.% to 10.0 wt.% of the metal oxide catalyst component.

7. The process of any one of claims 1 to 6, wherein the mixed metal oxide catalyst component comprises a metal oxide support material comprising zirconia.

8. The process of claim 7, wherein the metal oxide catalyst component comprises gallium supported on zirconia.

9. The process of any one of claims 1 to 8, wherein the reaction zone operates at a temperature from 300 °C to 500 °C.

10. The process of any one of claims 1 to 9, wherein the reaction zone operates at a temperature from 400 °C to 470 °C.

11. The process of any one of claims 1 to 10, wherein the reaction zone operates at a pressure from 2000 kPa (20 bar) to 7000 kPa (70 bar).

12. The process of any one of claims 1 to 11, wherein the gas hourly space velocity (GHSV) is greater than 500 $hr^{-1}$.

13. The process of any one of claims 1 to 12, wherein the hybrid catalyst comprises a metal oxide catalyst component to microporous catalyst component ratio of from 1:1 to 10:1.

14. The process of any one of claims 1 to 13, wherein the $C_2/C_3$ molar ratio of the product stream is greater than or equal to 1.0.

15. The process of any one of claims 1 to 14, wherein the $C_2$ to $C_3$ hydrocarbons consist essentially of olefins.


**Patentansprüche**

1. Verfahren zum Herstellen von $C_2$-bis $C_3$-Kohlenwasserstoffen, umfassend:

Einbringen eines Zufuhrstroms, umfassend Wasserstoffgas und ein kohlenstoffhaltiges Gas, die aus der Gruppe ausgewählt sind, bestehend aus Kohlenmonoxid, Kohlendioxid und Mischungen davon, in eine Reaktionszone eines Reaktors; und
Umwandeln des Zufuhrstroms in einen Produktstrom, umfassend $C_2$- bis $C_3$-Kohlenwasserstoffe in der Reaktionszone in der Gegenwart eines Hybridkatalysators, der Hybridkatalysator umfassend:

eine gemischte Metalloxidkatalysatorkomponente; und
eine mikroporöse Katalysatorkomponente, die 8-MR-Porenöffnungen umfasst, wobei:

die mikroporöse Katalysatorkomponente aus einem natürlichen Mineral abgeleitet ist; und
der Produktstrom eine kombinierte $C_2$ und $C_3$-Selektivität von mehr als 40 C-Mol-% in dem Produktstrom umfasst.

**2.** Verfahren nach Anspruch 1, wobei die mikroporöse Katalysatorkomponente eine Chabazit-, Erionit- oder Levyne-Struktur aufweist.

**3.** Verfahren nach Anspruch 1, wobei die mikroporöse Katalysatorkomponente eine Heulandit-, Phillipsit-, Stilbit- oder Natrolit-Struktur umfasst.

**4.** Verfahren nach einem der Ansprüche 1 bis 3, wobei die mikroporöse Katalysatorkomponente ein $SiO_2/Al_2O_3$-Molverhältnis von weniger als oder gleich 50,0 umfasst.

**5.** Verfahren nach einem der Ansprüche 1 bis 3, wobei die mikroporöse Katalysatorkomponente ein $SiO_2/Al_2O_3$-Molverhältnis von weniger als oder gleich 10,0 umfasst.

**6.** Prozess nach einem der Ansprüche 1 bis 5, wobei die Metallkomponente von 0,1 Gew.-% bis 10,0 Gew.-% die Metalloxidkatalysatorkomponente umfasst.

**7.** Verfahren nach einem der Ansprüche 1 bis 6, wobei die gemischte Metalloxidkatalysatorkomponente ein Metalloxid-Trägermaterial umfasst, das Zirkoniumdioxid umfasst.

**8.** Verfahren nach Anspruch 7, wobei die Metalloxidkatalysatorkomponente Gallium umfasst, das auf Zirkoniumdioxid getragen wird.

**9.** Verfahren nach einem der Ansprüche 1 bis 8, wobei die Reaktionszone bei einer Temperatur von 300 °C bis 500 °C betrieben wird.

**10.** Verfahren nach einem der Ansprüche 1 bis 9, wobei die Reaktionszone bei einer Temperatur von 400 °C bis 470 °C betrieben wird.

**11.** Verfahren nach einem der Ansprüche 1 bis 10, wobei die Reaktionszone bei einem Druck von 2000 kPa (20 bar) bis 7000 kPa (70 bar) betrieben wird.

**12.** Verfahren nach einem der Ansprüche 1 bis 11, wobei die stündliche Gasraumgeschwindigkeit (GHSV) größer als 500 $h^{-1}$ ist.

**13.** Verfahren nach einem der Ansprüche 1 bis 12, wobei der Hybridkatalysator ein Verhältnis von Metalloxidkatalysatorkomponente zu mikroporöser Katalysatorkomponente von 1:1 bis 10:1 umfasst.

**14.** Verfahren nach einem der Ansprüche 1 bis 13, wobei das $C_2/C_3$-Molverhältnis des Produktstroms größer als oder gleich 1,0 ist.

**15.** Verfahren nach einem der Ansprüche 1 bis 14, wobei die $C_2$- bis $C_3$-Kohlenwasserstoffe im Wesentlichen aus Olefinen bestehen.

**Revendications**

**1.** Procédé permettant de préparer des hydrocarbures en $C_2$ à $C_3$, comprenant :

l'introduction d'un débit d'entrée comprenant de l'hydrogène gazeux et un gaz contenant du carbone choisi dans le groupe constitué de monoxyde de carbone, dioxyde de carbone, et mélanges de ceux-ci dans une zone de réaction d'un réacteur ; et
la conversion du débit d'entrée en un débit diluat comprenant des hydrocarbures en $C_2$ à $C_3$ dans la zone de réaction en présence d'un catalyseur hybride, le catalyseur hybride comprenant :

un composant de catalyseur à base d'oxyde métallique mixte ; et
un composant de catalyseur microporeux comprenant des ouvertures de pore 8-MR, dans lequel :

le composant de catalyseur microporeux est dérivé d'un minéral naturel ; et
le débit diluat comprend une sélectivité à $C_2$ et $C_3$ combinée supérieure à 40 % en moles dans le débit diluat.

2. Procédé selon la revendication 1, dans lequel le composant de catalyseur microporeux comprend une structure de chabazite, érionite, ou levyne.

3. Procédé selon la revendication 1, dans lequel le composant de catalyseur microporeux comprend une structure d'heulandite, phillipsite, stilbite, ou natrolite.

4. Procédé selon l'une quelconque des revendications 1 à 3, dans lequel le composant de catalyseur microporeux comprend un rapport molaire $SiO_2/Al_2O_3$ inférieur ou égal à 50,0.

5. Procédé selon l'une quelconque des revendications 1 à 3, dans lequel le composant de catalyseur microporeux comprend un rapport molaire $SiO_2/Al_2O_3$ inférieur ou égal à 10,0.

6. Procédé selon l'une quelconque des revendications 1 à 5, dans lequel le composant métallique comprend de 0,1 % en poids à 10,0 % en poids du composant de catalyseur à base d'oxyde métallique.

7. Procédé selon l'une quelconque des revendications 1 à 6, dans lequel le composant de catalyseur à base d'oxyde métallique mixte comprend un matériau de support d'oxyde métallique comprenant de la zircone.

8. Procédé selon la revendication 7, dans lequel le composant de catalyseur à base d'oxyde métallique comprend du gallium supporté sur de la zircone.

9. Procédé selon l'une quelconque des revendications 1 à 8, dans lequel la zone de réaction fonctionne à une température allant de 300 °C à 500 °C.

10. Procédé selon l'une quelconque des revendications 1 à 9, dans lequel la zone de réaction fonctionne à une température allant de 400 °C à 470 °C.

11. Procédé selon l'une quelconque des revendications 1 à 10, dans lequel la zone de réaction fonctionne à une pression de 2000 kPa (20 bar) à 7000 kPa (70 bar).

12. Procédé selon l'une quelconque des revendications 1 à 11, dans lequel la vitesse spatiale horaire du gaz (GHSV) est supérieure à 500 $h^{-1}$.

13. Procédé selon l'une quelconque des revendications 1 à 12, dans lequel le catalyseur hybride comprend un rapport composant de catalyseur à base d'oxyde métallique au composant de catalyseur microporeux allant de 1:1 à 10:1.

14. Procédé selon l'une quelconque des revendications 1 à 13, dans lequel le rapport molaire $C_2/C_3$ du débit diluat est supérieur ou égal à 1,0.

15. Procédé selon l'une quelconque des revendications 1 à 14, dans lequel les hydrocarbures en $C_2$ à $C_3$ sont constitués sensiblement d'oléfines.

FIG. 1

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2020005703 A
- US 4503023 A
- US 4440871 A

### Non-patent literature cited in the description

- Minerals Yearbook. U.S. Department of the Interior, August 2018
- Zeolites. IHS Chemical Economic Handbook Report. 16 December 2016
- Atlas of zeolite framework types. Elsevier, 2007, 381-86
- *ACS Catalysis,* 2019, vol. 9, 6017
- **JOHN HUN KANG ; FAISAL H. ALSHAFEI ; STACEY I. ZONES ; MARK E. DAVIS.** Cage-Defining Ring: A Molecular Sieve Structural Indicator for Light Olefin Product Distribution from the Methanol-to-Olefins Reaction. *ACS Catalysis,* 11 April 2019, vol. 9 (7), 6012-6019
- *Chemical Communications,* 2016, vol. 42, 3227